# EUROPEAN PATENT SPECIFICATION

(11) **EP 0 784 603 B1**
(45) Date of publication and mention of the grant of the patent: **23.04.2003**
(21) Application number: 95933510.0
(22) Date of filing: 09.10.1995
(51) Int. Cl.: C07B 55/00, C07D 211/60

(54) **RACEMISATION OF PRECURSORS TO LEVOBUPIVACAINE AND ANALOGUES THEREOF**
RACEMISIERUNG VON LEVOBUPIVACAINE-VORLÄUFERN UND IHREN ANALOGEN
RACEMISATION DE PRECURSEURS DE LEVOBUPIVACAINE ET ANALOGUES DESDITS PRECURSEURS

(30) Priority: 07.10.1994 GB 9420245; 10.03.1995 GB 9504924
(43) Date of publication of application: 23.07.1997
(73) Proprietor: Darwin Discovery Limited, Slough Berks SL1 3WE (GB)
(72) Inventor: DYER, Ulrich, Conrad, Chiroscience Limited, Cambridge CB4 4WE (GB); McCAGUE, Raymond, Cambridgeshire CB4 6EE (GB); WOODS, Martin, Chiroscience Limited, Cambridge CB4 4WE (GB)
(74) Representative: Perry, Robert Edward
(86) International application number: GB9502383
(87) International publication number: WO96011173

(56) References cited:
- EP-A- 0 137 371
- EP-A- 0 173 921
- EP-A- 0 175 840
- FR-A- 2 074 022
- BULLETIN OF THE CHEMICAL SOCIETY OF JAPAN, vol. 54, no. 11, November 1981 TOKYO, JP, pages 3286-90, C. HONGO ET. AL. 'Asymmetric Transformation of N-Acyl-DL-Amino Acids.' cited in the application
- JOURNAL OF THE AMERICAN CHEMICAL SOCIETY, vol. 111, no. 16, 2 August 1989 WASHINGTON DC, US, pages 6354-64, H. K. CHENAULT ET. AL. 'Kinetic Resolution of Unnatural and Rarely Occurring Amino Acids: Enantioselective Hydrolysis of N-acyl Amino Acids Catalysed by Acylase I.'

## Description

### Field of Invention

This invention relates to a process for the racemisation of N-acylamino-acids.

### Background of the Invention

N-acylamino-acids are useful in the pharmaceutical industry. Processes for their racemisation are known; see Bull. Chem. Soc. Jpn. 60:649-652 (1987), 56:3744-3747 (1983), 60:4321-4323 (1987), 66:965-970 (1992), 66:2430-1437 (1992), and 64:191-195 (1991); and Agr. Biol. Chem. 43:395 (1979).

The known processes generally require either specialised equipment or the use of reagents which are not applicable to industrial processes. For example, Hongo et al, Bull. Chem. Soc. Jpn. 54:3386 (1981), describes the use of chloroform and acetic anhydride for the racemisation of N-butanoyl-proline. This would clearly have a high environmental impact if carried out on a commercial scale.

EP-A-0137371 describes the racemisation of N-acylamino-acids, by heating with a carboxylic acid such as benzoic acid or phenylacetic acid. The use of catalytic amounts of acid and an inert solvent are disclosed but not preferred.

### Summary of the Invention

The present invention is based on the surprising discovery that dehydrating conditions, using a catalytic amount of 4-toluenesulphonic acid in an organic solvent, allow N-acylpipecolic acids to racemise, e.g. from optically-enriched to racemic form.

### Description of the Invention

A preferred embodiment of the invention utilises a catalytic amount of 4-toluenesulphonic acid in toluene. Without wishing to be bound by theory, it may be that the reaction of the invention proceeds via an azlactone intermediate.

Alternatively, an orthoester such as orthoformate or orthoacetate, e.g. trimethyl orthoacetate, can be used, introducing the COR group in the reaction. Thus, the starting material may be N-unsubstituted, e.g. R²HN-CHR¹-COOH (optically-active) can be converted to R-CO-NR²-CHR¹-COOH (racemate). The reagent is preferably used in a stoichiometric amount. This reaction may be conducted with azeotropic removal of water.

The starting material may be prepared by any suitable resolution process, examples of which are well known to those skilled in that art. A particularly useful biotransformation process is described in WO-A-9510604.

An important aspect of this invention relates to the ability to operate the process on an industrial scale. This in turn means that the optically-enriched N-acylpipecolic acids themselves, e.g. obtained by resolution but to an extent that may be insufficiently enantiopure for immediate use, become useful products. This applies particularly to mixtures of enantiomers in which one, often the (*R*)-enantiomer, is present in an enantiomeric excess of 20 to 80%, preferably 25 to 75%, more preferably 30 to 70%, and most preferably 35 to 65%, with respect to its optical antipode. For example, a mixture enriched in the (*R*)-enantiomer can be used practically, by racemisation and subsequent resolution. The desired product, e.g. (*S*)-N-acylpipecolic acid, can be converted to levobupivacaine by methods that will be evident to those skilled in the art.

Crystallisation to a pure racemate and possible dynamic resolutions with chiral salts are other benefits and applications. N-acylpipecolic acids are useful in the synthesis of analgesics such as levobupivacaine and ropivicaine, e.g. by racemisation of an optically-enriched mixture, resolution, reaction with 2,6-dimethylaniline and N-alkylation. All there reactions may be conducted by known methodology.

The following Example illustrates the invention.

### Example

(*R*)-N-acetylpipecolic acid was placed in 10 volumes of toluene and heated, with stirring, to reflux. On attaining reflux, a catalytic amount of 4-toluenesulphonic acid was added to the solution which was then left to reflux for two hours with stirring. After this time had elapsed, the toluene was removed by rotary evaporation. To the residual solid was added 10 volumes of distilled water; this was then extracted with methyl ethyl ketone (MEK) (3 x 10 vols) to leave the sulphonic acid in the aqueous layer with any pipecolic acid formed. The MEK extracts were then concentrated down to a solid on the rotary evaporator to give racemic N-acetylpipecolic acid, with 97 % recovery.

## Claims

1. A process for racemising an optically-active N-acylamino-acid, which comprises heating the N-acylamino-acid with a catalytic amount of an organic acid, in an inert solvent, under dehydrating conditions, **characterised in that** the organic acid is 4-toluenesulphonic acid and that the N-acylamino-acid is a N-acylpipecolic acid.

2. A process for preparing levobupivacaine, which comprises resolution of a racemate of a N-acylpipecolic acid, separating the (*S*)-N-acylpipecolic-acid and converting it to levobupivacaine by reaction with 2,6-dimethylaniline and alkylation with a butylating agent; and racemising the (*R*)-N-acylpipecolic acid by a process according to claim 1.

3. A process for preparing ropivacaine, which comprises resolution of a racemate of a N-acylpipecolic acid, separating the (*S*)-N-acylpipecolic-acid and converting it to ropivacaine by reaction with 2,6-dimethylaniline and alkylation with a propylating agent; and racemising the (*R*)-N-acylpipecolic acid by a process according to claim 1.

## Patentansprüche

1. Verfahren zum Racemisieren einer optisch aktiven N-Acylaminosäure, welches umfasst das Erwärmen der N-Acylaminosäure mit einer katalytischen Menge einer organischen Säure in einem organischen Lösungsmittel unter dehydratisierenden Bedingungen, **dadurch gekennzeichnet, dass** die organische Säure 4-Toluolsulfonsäure ist und die N-Acylaminosäure eine N-Acylpipecolinsäure ist.

2. Verfahren zur Herstellung von Levobupivacain, welches umfasst die Aufspaltung eines Racemats einer N-Acylpipecolinsäure, das Abtrennen der (S)-N-Acylpipecolinsäure und deren Überführung in Levobupivacain durch Umsetzen mit 2,6-Dimethylanilin und Alkylierung mit einem Butylierungsmittel und das Racemisieren der (R)-N-Acylpipecolinsäure durch ein Verfahren nach Anspruch 1.

3. Verfahren zur Herstellung von Ropivacain, welches umfasst die Aufspaltung eines Racemats einer N-Acylpipecolinsäure, das Abtrennen der (S)-N-Acylpipecolinsäure und deren Überführung in Ropivacain durch Umsetzen mit 2,6-Dimethylanilin und Alkylierung mit einem Propylierungsmittel und das Racemisieren der (R)-N-Acylpipecolinsäure durch ein Verfahren nach Anspruch 1.

## Revendications

1. Procédé pour racémiser un N-acylamino-acide optiquement actif, qui comprend le chauffage du N-acylamino-acide avec une quantité catalytique d'un acide organique, dans un solvant inerte, dans des conditions déshydratantes, **caractérisé en ce que** l'acide organique est l'acide toluène-4-sulfonique et que le N-acylamino-acide est un acide N-acylpipécolique.

2. Procédé pour préparer la lévobupivacaïne, qui comprend la résolution d'un racémate d'un acide N-acylpipécolique, la séparation de l'acide (S)-N-acylpipécolique et la transformation de celui-ci en lévobupivacaïne par réaction avec la diméthyl-2,6-aniline et l'alkylation avec un agent butylant; et la racémisation de l'acide (R)-N-acylpipécolique par le procédé décrit dans la revendication 1.

3. Procédé pour préparer la ropivacaïne, qui comprend la résolution d'un racémate d'un acide N-acylpipécolique, la séparation de l'acide (S)-N-acylpipécolique et la transformation de celui-ci en ropivacaïne par réaction avec la diméthyl-2,6-aniline et l'alkylation avec un agent propylant; et la racémisation de l'acide (R)-N-acyipipécolique par le procédé de la revendication 1.
